# EUROPEAN PATENT SPECIFICATION

(11) **EP 2 077 896 B1**
(45) Date of publication and mention of the grant of the patent: **31.07.2013**
(21) Application number: 07853587.9
(22) Date of filing: 21.09.2007
(51) Int. Cl.: A61N 1/37, A61N 1/05, A61N 1/08, A61N 1/16, A61N 1/362

(54) **RADIOFREQUENCY (RF)-SHUNTED SLEEVE HEAD AND USE IN ELECTRICAL STIMULATION LEADS**
HÜLSENKOPF MIT HOCHFREQUENZ (HF) SHUNT UND VERWENDUNG IN ELEKTRISCHEN STIMULATIONSLEITUNGEN
TETE DE MANCHON SHUNTEE PAR RADIOFREQUENCES (RF) ET SON UTILISATION DANS DES FILS DE STIMULATION ELECTRIQUE

(30) Priority: 21.09.2006 US 826476 P
(43) Date of publication of application: 15.07.2009
(73) Proprietor: MEDTRONIC, INC., Minneapolis, Minnesota 55432 (US)
(72) Inventor: REINKE, James, D., Maple Grove, Minnesota 55311 (US); YANG, Zhongping, Woodbury, Minnesota 55129 (US); SOMMER, John, L., Coon Rapids, Minnesota 55448 (US)
(74) Representative: Hughes, Andrea Michelle
(86) International application number: PCT/US2007/079112
(87) International publication number: WO 2008/036865

(56) References cited:
- WO-A-03/063946
- US-A1- 2005 070 972

## Description

The invention relates to medical devices and, more particularly, to implantable medical device leads for use with implantable medical devices (IMDs).

### BACKGROUND

In the medical field, implantable leads are used with a wide variety of medical devices. For example, implantable leads are commonly used to form part of implantable cardiac pacemakers that provide therapeutic stimulation to the heart by delivering pacing, cardioversion or defibrillation pulses. The pulses can be delivered to the heart via electrodes disposed on the leads, e.g., typically near distal ends of the leads. In that case, the leads may position the electrodes with respect to various cardiac locations so that the pacemaker can deliver pulses to the appropriate locations. Leads are also used for sensing purposes, or for both sensing and stimulation purposes. Implantable leads are also used in neurological devices, muscular stimulation therapy, and devices that sense chemical conditions in a patient's blood, gastric system stimulators.

Occasionally, patients that have implantable leads may benefit from a magnet resonance image being taken of a particular area of his or her body. Magnetic resonance imaging (MRI) techniques achieve a more effective image of the soft tissues of the heart and vascular system. MRI procedures can also image these features without delivering a high dosage of radiation to the body of the patient, and as a result, MRI procedures may be repeated reliably and safely. However, MRI devices may operate at frequencies of 10 megahertz or higher, which may cause energy to be transferred to the lead. In particular, the high frequency fields induce a voltage in the lead, causing the potential of the lead to be higher than the surrounding tissue. In effect, the lead behaves as an antenna. Current may flow from the electrode into the tissue proximate to the electrode due to induced voltage. It is therefore desirable to develop a lead that addresses this disadvantage.

WO 03/063946 relates to a method and apparatus for providing electrical stimuli to tissue or receiving electrical stimuli corresponding to one or more conditions in tissue.

The present invention provides a medical lead as defined in claim 1.

### BRIEF DESCRIPTION OF DRAWINGS

Aspects and features of the present invention will be appreciated as the same becomes better understood by reference to the following detailed description of the embodiments of the invention when considered in connection with the accompanying drawings, wherein:
FIG. 1 is a conceptual perspective view of a medical device system including a medical device coupled to a lead according to an embodiment of the present invention;
FIG. 2 is a cross-sectional view of an electrode assembly located at a distal end of a medical lead;
FIG. 3 depicts multiple layers of insulating material over a conductive element of the electrode assembly depicted in FIG. 2;
FIG. 4A depicts a cross-sectional view of a conductive ring coupled to a conductive sealer for the electrode assembly depicted in FIG. 2;
FIG. 4B depicts a top view of a conductive ring coupled to a conductive sealer for the electrode assembly depicted in FIG. 2;
FIG. 4C depicts a cross-sectional view of conductive rings and a conductive sealer coupled to a shaft;
FIG. 4D depicts an angled view of a conductive sealer;
FIG. 5 is a schematic diagram of a simplified circuit for a medical device system under pacing and sensing conditions;
FIG. 6 is a schematic diagram of another simplified circuit for a medical device system under magnetic resonance imaging conditions;
FIG. 7 is a block diagram of an electrochemical impedance spectrum (EIS) measurement system;
FIG. 8 graphically depicts an EIS measurement which shows an impedance spectrum and the frequency corresponding to typical pacing conditions;
FIG. 9 graphically depicts reduced heating in the tip of the medical lead; and
FIG. 10 is a flow diagram that depicts the method of producing an electrode assembly.

### DETAILED DESCRIPTION

The present invention is directed to a medical lead. The medical lead of the present invention includes a radio frequency signal (RF) shunted sleeve head (also referred to as a capacitive shunt). The RF shunted sleeve head is coupled to a lead body and to the tip electrode. The RF shunted sleeve head preferably comprises a biostable dielectric coating introduced over the conductive element.

The medical lead is able to effectively manage high frequency signals from other devices such that the operation of a medical lead is not detrimentally affected. For example, the electrode assembly of the medical lead shunts the high frequency RF signals (e.g. 21megaHertz (Mhz) to 128MHz) generated from a magnetic resonance imaging (MRI) machine away from the tip electrode and into the larger area of the RF shunted sleeve head. This in turn, reduces the current density in the tissue near the electrode and reduces the level of heating. Consequently, a patient with a medical lead may undergo an MRI procedure without significantly affecting the operation of the medical lead.

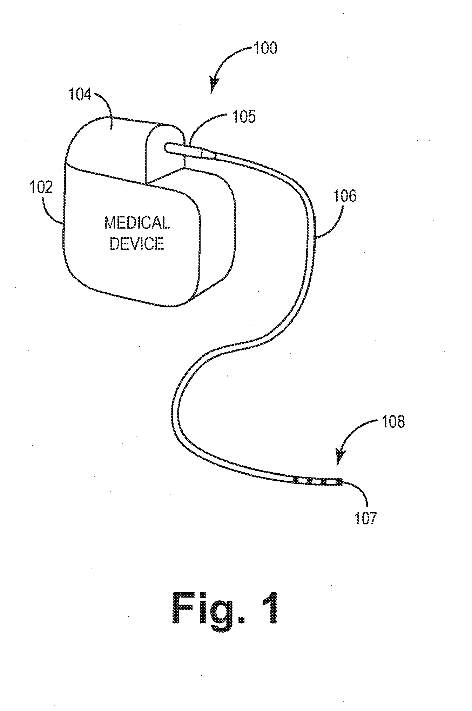

FIG. 1 depicts a medical device system 100. A medical device system 100 includes a medical device housing 102 having a connector module 104 that electrically couples various internal electrical components of medical device housing 102 to a proximal end 105 of a medical lead 106 (also referred to as a MRI/RF shunted lead, or a shunted lead). A medical device system 100 may comprise any of a wide variety of medical devices that include one or more medical lead(s) 106 and circuitry coupled to the medical lead(s) 106. An exemplary medical device system 100 may take the form of an implantable cardiac pacemaker, an implantable cardioverter, an implantable defibrillator, an implantable cardiac pacemaker-cardioverter-defibrillator (PCD), a neurostimulator, or a muscle stimulator. Medical device system 100 may deliver, for example, pacing, cardioversion or defibrillation pulses to a patient via electrodes 108 disposed on distal ends 107 of one or more lead(s) 106. In other words, lead 106 may position one or more electrodes 108 with respect to various cardiac locations so that medical device system 100 can deliver pulses to the appropriate locations.

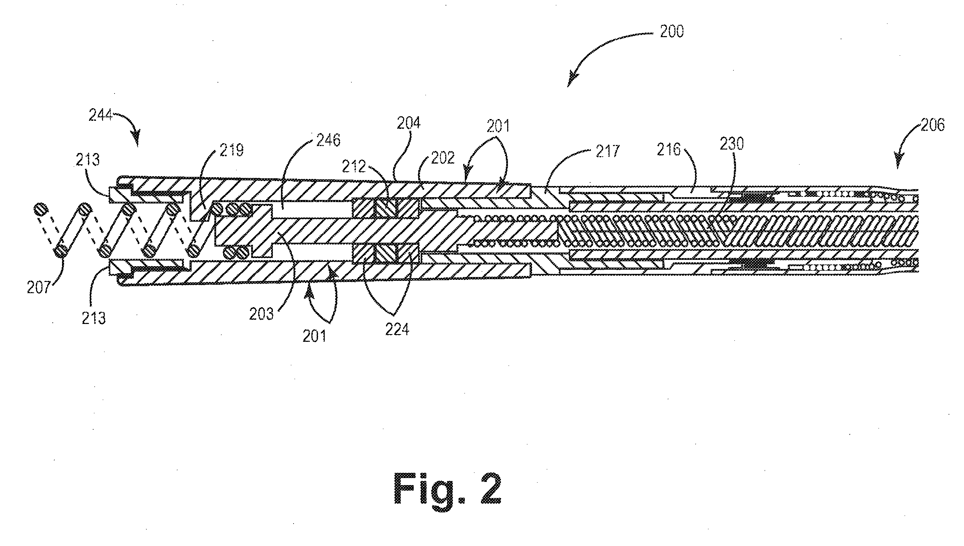

FIG. 2 depicts an electrode assembly 200 of a medical lead 106. Electrode assembly 200 includes a RF-shunted sleeve head 201 coupled to an electrode 207 (also referred to as a tip electrode), a monolithic controlled-release device (MCRD) 213, a conductive electrode shaft 203, a conductive sealer 212, conductive rings 224, a ring electrode 216, and a non-conductive spacer 217. At a distal end 244 of electrode assembly 200, a sharpened distal tip (not shown) facilitates fixation of the distal end of helically shaped electrode 207 into tissue of a patient. The proximal end of electrode 207 is securely seated between MCRD 213, electrode shaft 203, and a securing member 219 that protrudes from an inner diameter of RF shunted sleeve head 201. MCRD 213 provides chronic steroid elution to maintain a low pacing threshold for a medical device system 100.

RF-shunted sleeve head 201 is electrically connected to a conductive electrode shaft 203 via two parallel conductive rings 224 (e.g. C-rings etc.) and a conductive sealer 212 (also referred to as a sealing washer). At a proximal end 206 of electrode assembly 200, coil 230 is electrically coupled to conductive electrode shaft 203. RF shunted sleeve head 201 comprises a conductive element 202 surrounded or at least partially covered by an insulating material 204 (also referred to as a dielectric material). In one embodiment, conductive element 202 is cylindrically shaped (e.g. ring, etc.) or may possess other suitable shapes. Exemplary dimensions for conductive element 202 include a diameter of about 6.5 French (Fr.) by about 9 millimeters (mm) in length, an outer diameter of about 82mils and an inner diameter of about 62mils. Conductive element 202, in one embodiment, includes an increased diameter at the distal end and a reduced diameter at the proximal end of the conductive element 202. The surface area of conductive element 202 is about 60mm² which is much larger than the 5.5mm² surface area of electrode 207. A large surface area ratio, defined by the ratio of the surface area of conductive element 202 to the surface area of electrode 207, is desired to insure that current induced from a MRI machine is spread over a large area of electrode assembly 200. A tenfold (i.e. 10X) larger surface area ratio results in about tenfold lower temperatures at the tip of electrode 207 assuming ring electrode 216 has low impedance at high frequencies. Conductive element 202 comprises materials that are chemically stable, biocompatible, and x-ray transparent. Exemplary material used to form conductive element 202 includes titanium, titanium alloy, conductive polymers, and/or other suitable materials.

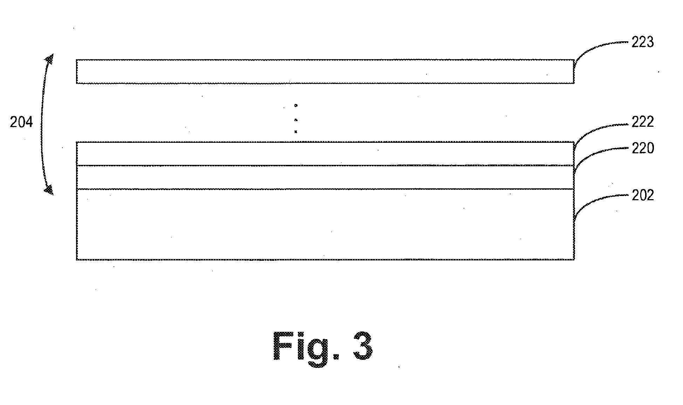

Referring to Fig. 3, insulative material 204 may be formed from a single layer or multiple layers such as first layer 220, second layer 222, and N layer 223, where N is a whole number that is less than 100, and is typically less than about 30 layers. Each layer may comprise different insulating materials, two or more different insulating materials, or the same insulating materials. Insulative material 204 includes a thickness from about 1 nanometer (nm) to about 1 millimeter (mm)) and extends from about 1 mm to about 20 mm along the length of conductive element 202. Insulative material 204 may be formed from any of a wide variety of insulating materials. Exemplary insulating material include at least one or more of Parylene, polyamide, metal oxides, polyimide, urethane, silicone, tetrafluroethylene (ETFE), polytetrafluroethylene (PTFE), or the like. Parylene is the preferred insulating material 204. The preferred Parylene is Parylene C. Parylene C is formed through a dimer vacuum deposition process. The dimer is commercially available from Specialty Coating Systems located in Clear Lake, Wisconsin. Numerous techniques may be employed to introduce insulating material 204 over the outside of sleeve head 201 and/or partially inside sleeve head 201. Exemplary techniques include chemical vapor deposition, dip coating, or thermal extrusion.

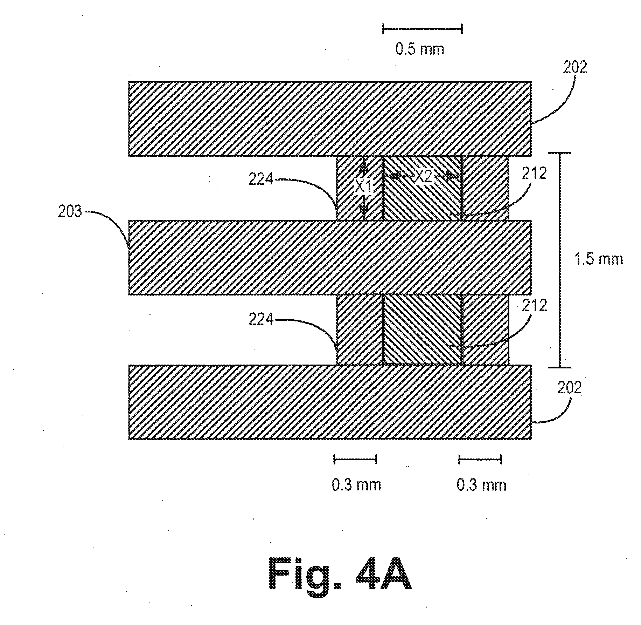

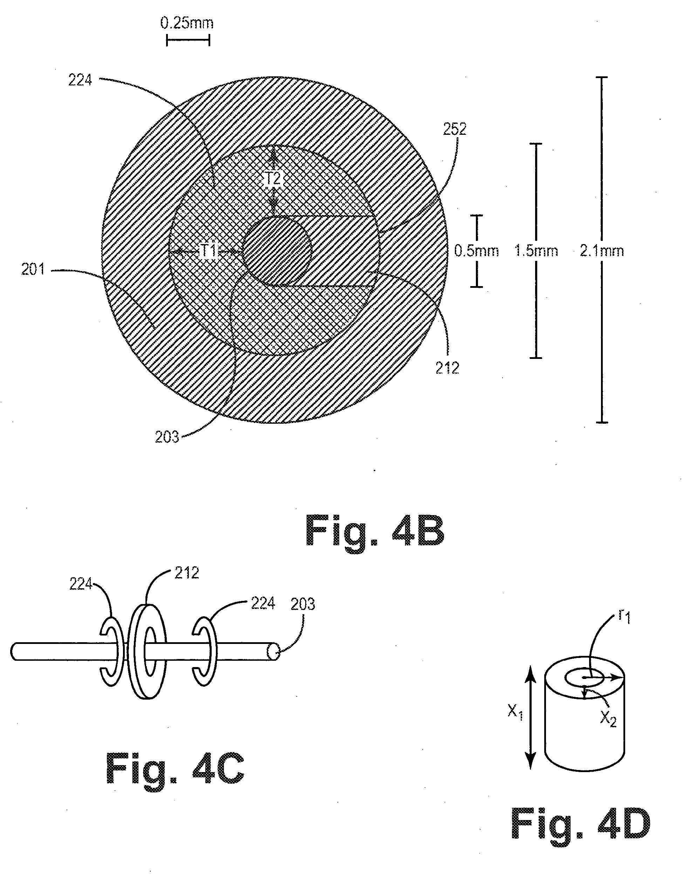

Conductive sealer 212 conducts current and also prevents fluid from passing through lumen 246. Referring to FIGs. 4A-4D, conductive sealer 212 is substantially ring (i.e. o-ring) or disk shaped but other suitable shapes may also be employed. In one embodiment, conductive sealer 212 is defined by X1, X2 and radius (r1). X1 ranges from about 0.1mm to about 0.50 mm, X2 extends from about 0.1mm to about 1.0 mm, and r1 extends from about 0.5mm to about 1.0mm. Curved end 252 extends to about 1.25mm from the center of shaft 203 and includes a curve defined by a radius of about 0.5mm.

Conductive sealer 212 comprises a polymer and a conductive polymer such as a conductive powder (e.g. carbon, carbon nanotube, silver, platinum etc.). The conductive polymer ranges from about 1% to about 25% of conductive sealer 212. The polymer (e.g. silicone etc.) is commercially available from Nusil Technology LLC, located in Carpinteria, California. Polyurethane is commercially available from The Polymer Technology Group Inc. located in Berkeley, California.

Conductive rings 224 are shaped, in one embodiment, as a C-ring to receive conductive sealer 212. Conductive rings 224 have an outer diameter of about 1.5mm, an inner diameter of about 0.7mm, and a thickness that ranges from about 0.25 mm (T1) to about 0.5 mm (T2). Conductive rings 224 are comprised of platinum or other suitable materials.

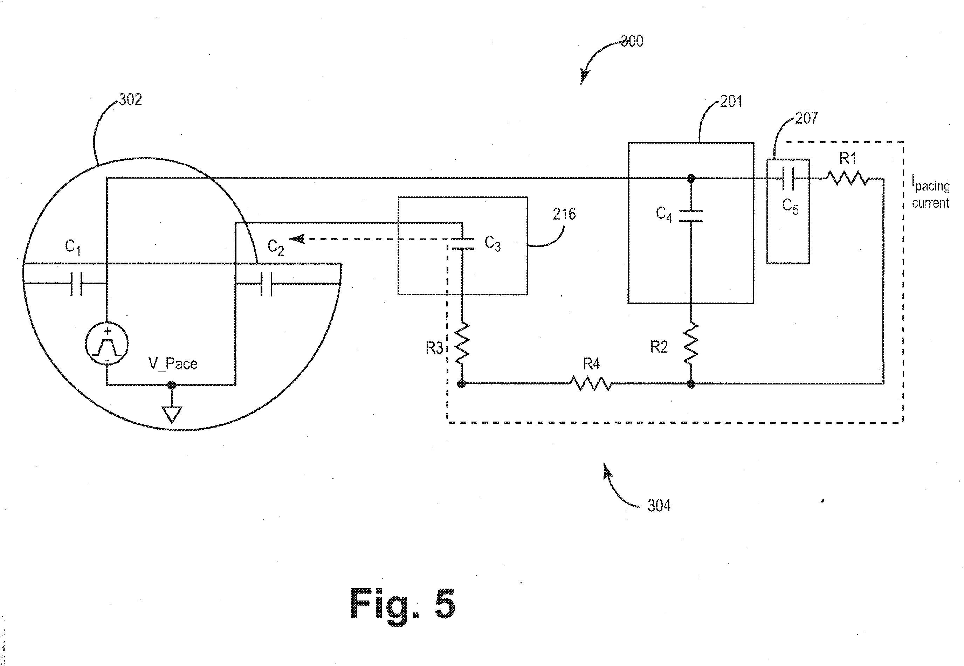

FIG. 5 depicts a simplified circuit 300 for a medical device system 100 during normal pacing conditions. Pacing conditions typically involve low frequency signals (e.g. 1000Hz). Circuit 300 includes an implantable medical device (IMD) circuit 302 (e.g. a pacemaker circuit, neurostimilator circuit etc.) connected to a bipolar shunted lead circuit 304. IMD circuit 302 comprises two filter capacitors C1 and C2 connected to housing 102. C1 and C2 filter high frequency electromagnetic interference (EMI) so that high frequency signals from a MRI machine do not affect the sensing operation of medical lead 106. Exemplary values for C1 is about 1 to 10 nanoFarad (nF) and C2 is 1-10nF.

Bipolar shunted lead circuit 304 includes ring electrode 216, RF shunted sleeve head 201, and tip electrode 207. Capacitors C3, C4, and C5 correspond to ring electrode 216, sleeve head 201, and tip electrode 207, respectively. Resistors R1, R2, R3, and R4 represent the impedance created by tissue and/or blood of the patient. R1, R2, and R3 along with capacitors C5, C4, and C3 represent the electrode to tissue interface impedances. Generally, larger area electrodes result in larger values of capacitance and smaller values of resistance. However, the addition of an insulating material 204 over sleeve head 201 reduces the effect of electrode 207 to tissue interface impedances and its capacitance. In particular, C4 comprises a series capacitance of the electrode to tissue interface and capacitance due to insulation. Exemplary values for bipolar shunted lead circuit 304 include C3 at 10 microF (uF), R3 is 100 Ohm (Ω), R2 is 100 Ω, C5 is 1 uF, R1 is 500 Ω, and C4 is about 0.5 nanoFarad (nF) to about 10nF. Optimally, C4 should possess a capacitance of about 1-2 nF.

Generally, under typical pacing conditions, pacing current (I_{pacing current}) flows from tip electrode 207 to ring electrode 216 and then returns to IMD circuit 302. Negligible or no current I_{pacing current} passes through the RF shunted sleeve head 201 and resistor R2 because under a low frequency or direct current (DC) application, capacitor C4 acts like an open circuit to a constant voltage across its terminals. A portion of the I_{pacing current} passes to the patient's tissue, represented as resistor R1, due to the large capacitance of C5 associated with tip electrode 207. Similarly, a portion of the I_{pacing current} passes to the patient's tissue, represented as resistor R3, due to the large capacitance of C3 associated with ring electrode 216.

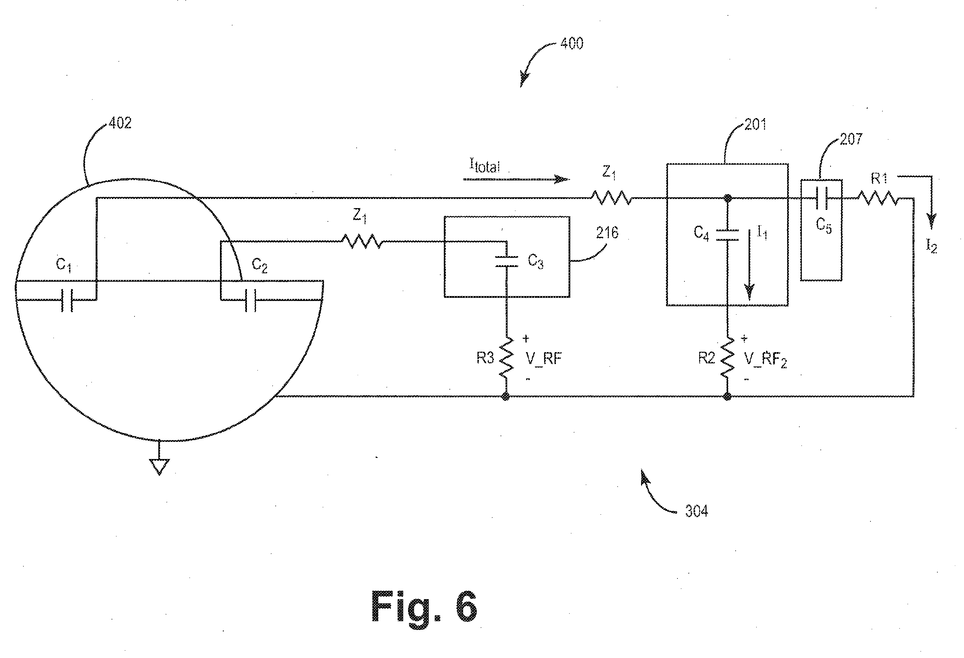

FIG. 6 depicts a simplified circuit 400 for a medical device system 100 during MRI conditions. Circuit 400 includes an IMD circuit 402 (e.g. a pacemaker circuit, neurostimulator circuit etc.) and a bipolar shunted lead 304. Circuit 400 includes the same elements as circuit 300, except lead resistance Z₁ is depicted between IMD circuit 402 and ring electrode 216 and also between IMD circuit 402 and RF shunted sleeve 201. Additionally, voltage potential differentiators (i.e. V-RF, V-RF2), are induced from a RF field.

Under MRI conditions, a large current (Iₜₒₜₐₗ) is induced in the medical lead 106 and IMD circuit 402 due to the RF voltage sources VRF and VRF2. A portion of the current, I₁, passes through the RF shunt sleeve head 201 represented by R2 and C4. Since the RF frequency is large, the impedance associated with C4 is small resulting in a large portion of the total current flowing through RF shunt sleeve head 201. The remainder of the current, I₂, passes through tip electrode 207, represented by capacitor C5. The current then returns to capacitor C2 of IMD circuit 402 through ring electrode 216 and through the body tissue and housing 102. Because conductive element 202 of RF shunted sleeve head 201 has a large surface area relative to tip electrode 207, the total current is spread over a larger total surface area resulting in a lower current density. This results in reduced local heating at the tip of electrode 207. In sum, RF shunted sleeve head 201 and tip electrode 207 cooperate to serve as a high-pass filter, allowing only high frequencies signals to "pass" through conductive element 202 and low frequency signals are blocked. RF shunted sleeve head 201 serves as a capacitor and passes the MRI high frequency current into the blood stream (represented by R2 in FIG. 6) surrounding sleeve head 201 rather than through tip electrode 207 and into the myocardium tissue (represented by R1).

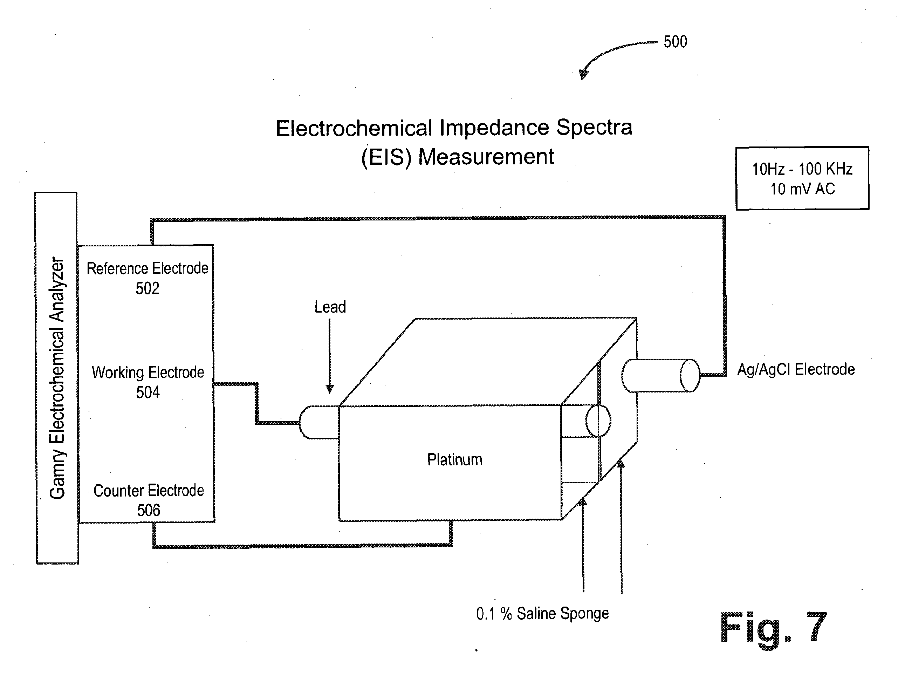

FIG. 7 is a block diagram of an electrochemical impedance spectrum (EIS) testing system 500 that assists in determining the optimal RF shunted sleeve head 201 for a medical lead 106. In particular, testing system 500 evaluates the electrochemical properties associated with RF shunted sleeve head 201. These electrochemical properties depend upon material(s) from which conductive element 202 and insulating material 204 are made. Testing system 500 includes a reference electrode 502, a tip electrode 504, and a counter electrode 506. RF shunted sleeve head 201 is evaluated by being inserted into a 0.1% saline soaked sponge located in a platinum container. Tip electrode 207 is not in the saline while ring electrode 216 is in the saline solution. Reference electrode 502, comprised of Ag/AgCl, is connected to the saline sponge. The performance of RF shunted sleeve head 201 is determined while it is exposed to signals at various frequencies between 100000 Hz and 0.01 HZ. Testing system 500 was used to optimize RF shunted sleeve head 201 for a medical lead 106.

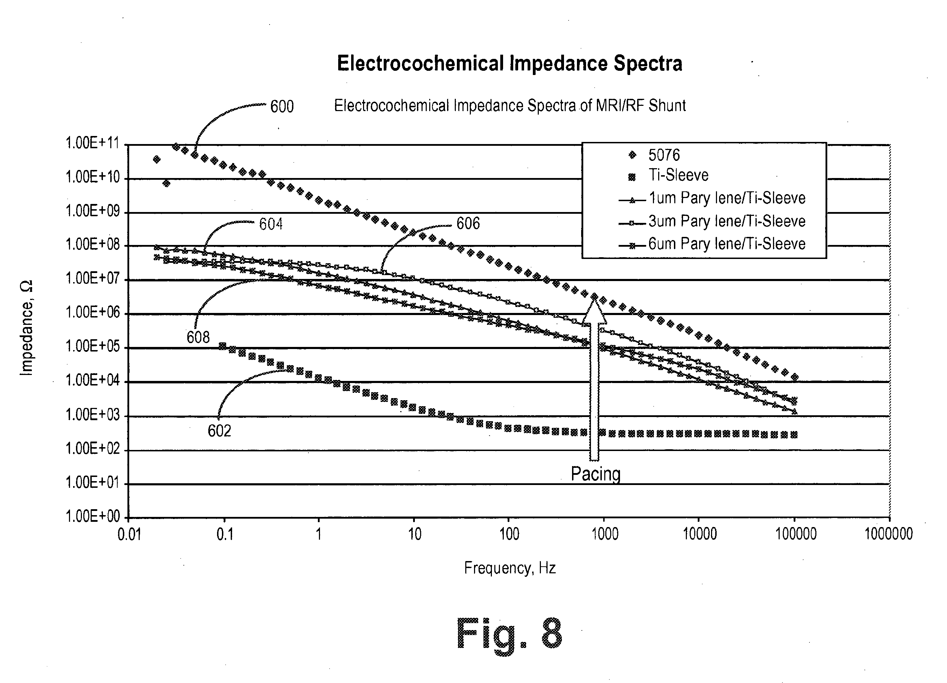

FIG. 8 graphically confirms that the RF shunted sleeve head 201 is able to achieve the desired capacitance that will result in heat reduction at electrode 207. The curves represent the impedance measured from tip electrode 207 conductor of lead 106 to the saline soaked sponge with tip electrode 207 out of the saline solution, but ring electrode 216 in the solution. RF shunted sleeve head 201 was exposed to signals of about 0.01 KHz to about 100KHz. A line 600 represents the impedance of sleeve head 201 on a standard lead. The impedance is high (e.g. 2.5 mega Ω) at typical pacing frequencies. The measured impedance is dominated by the capacitance from tip electrode 207 conductor to the ring electrode conductor due to the coaxial lead construction. A line 602 is the impedance of a bare titanium RF shunted sleeve head 201 with no insulating material 204. The impedance is very low, especially at high frequencies. Lines 604, 606, and 608, respectively represent the impedance of the different capacitive shunt which vary depending on the thickness of the parylene coating. The impedance is high at pacing and sensing frequencies (over 100kΩ). In general, thicker layers of insulating material 204 are expected to provide higher values of impedance. The discrepancy between the observed data and the predicted data can be caused by variations in the quality and consistency of insulating material 204 (e.g. Parylene dielectric coating etc.). This is primarily evident at the low frequency where the capacitance plays less of a role in setting the impedance.

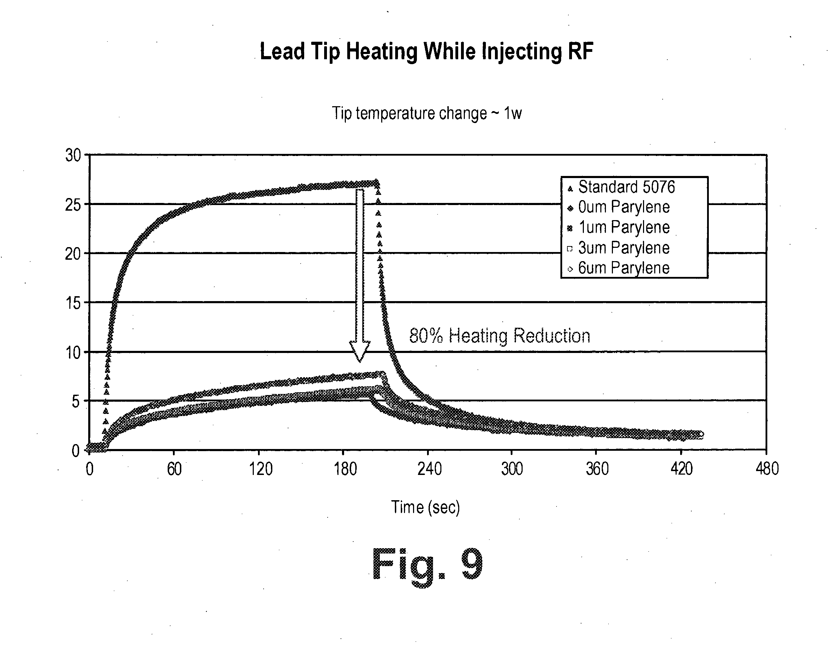

FIG. 9 graphically depicts that lead 106 is able to operate under MRI conditions. A significant drop in temperature rise exists with RF shunted sleeve head 201. For example, at least 80% of the RF power is shunted away from tip electrode 207 by sleeve head 201 which reduces the amount of heat at the electrode. Specifically, a standard lead exhibits peak heating at 27.0 Celcius (°C) after 3 minutes. For a titanium shunted lead with no insulation, peak heating is 5.6°C after 3 minutes. These results are achieved, in part, through a large effective surface area tip electrode at MRI frequencies (i.e. 21-128 megahertz (MHz) for 0.5-3T machines) and a normal effective surface area for pacing frequencies (i.e. 100Hz-10KHz) or sensing frequencies (i.e. 0.1-100hz). The large electrode area at MRI frequencies (1) creates a low impedance which reflects some of the energy away from the tip electrode 207 and back up the lead body, and (2) spreads the power over a larger surface area decreasing the peak temperature.

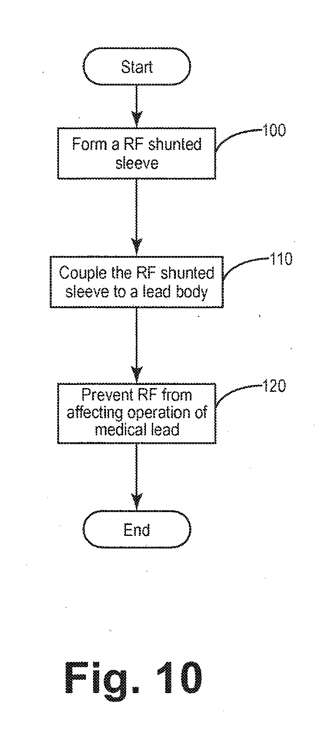

FIG. 10 is a flow diagram that depicts the method of producing a medical lead. At block 300, a RF shunted sleeve head is formed. The RF shunted sleeve head comprises a biostable dielectric coating introduced over the conductive element. At block 320, the RF shunted sleeve head is coupled to the lead body. At block 330, the RF is prevented from affecting the sensing operation of the medical lead.

It is understood that the present invention is not limited for use in pacemakers, cardioverters of defibrillators. Other uses of the leads described herein may include uses in patient monitoring devices, or devices that integrate monitoring and stimulation features. In those cases, the leads may include sensors disposed on distal ends of the respective lead for sensing patient conditions.

The leads described herein may be used with a neurological device such as a deep-brain stimulation device or a spinal cord stimulation device. In those cases, the leads may be stereotactically probed into the brain to position electrodes for deep brain stimulation, or into the spine for spinal stimulation. In other applications, the leads described herein may provide muscular stimulation therapy, gastric system stimulation, nerve stimulation, lower colon stimulation, drug or beneficial agent dispensing, recording or monitoring, gene therapy, or the like. In short, the leads described herein may find useful applications in a wide variety medical devices that implement leads and circuitry coupled to the leads.

Various embodiments of the invention have been described. These and other embodiments are within the scope of the following claims. For example, electrode 207 may include variously shaped electrodes such as ring shaped or other suitable shapes. Additionally, skilled artisans appreciate that other dimensions may be used for the mechanical and electrical elements described herein. Moreover, it is expected that 100% of the RF power is able to be shunted away by the RF sleeve head by implementing the claimed embodiment as well as other features.

## Claims

1. A medical device lead comprising:
a lead body,
a conductive electrode shaft within the lead body;
a coil conductor electrically coupled to the conductive electrode shaft;
a tip electrode (108) coupled to the conductive electrode shaft; and **characterised by**
a radio frequency (RF) shunted sleeve head (201) electrically coupled to the conductive electrode shaft; and wherein the RF shunted sleeve head includes:
a conductive element (202); and
a biostable tissue interfacing dielectric coating (204) introduced over the conductive element.

2. The medical device lead of claim 1, wherein the sleeve head is arranged to shunt high frequency signals away from the tip electrode and into the sleeve head.

3. The medical device lead of claim 1, wherein a surface area of the conductive element being about ten times larger than a surface area of the tip electrode.

4. The medical device lead of claim 1, wherein the conductive element includes an increased diameter at the distal end and a reduced diameter at the proximal end of the conductive element.

5. The medical device lead of claim 3, further comprising a conductive sealer (212) coupled to the conductive element.

6. The medical device lead of claim 1, the biostable dielectric coating being at least one of polyamide, parylene, and metal oxides.

7. The medical device lead of claim 1, the conductive element being at least one of titanium, titanium alloy, and conductive polymers.

8. The medical device lead of claim 2, wherein at least 80% of RF power is shunted away by the RF sleeve head.

9. The medical device lead of claim 2, wherein at least 70% of RF power is shunted away by the RF sleeve head.

10. The medical device lead of claim 2, wherein at least 60% of RF power is shunted away by the RF sleeve head.

11. The medical device lead of claim 1, further comprising a conductive sealer (212) that couples the RF shunted sleeve head to the conductive electrode shaft.

12. The medical device lead of any one of claims 1-11, further comprising one or more conductive rings (1224) that couple the RF shunted sleeve head to the conductive electrode shaft.

## Patentansprüche

1. Leitung einer medizinischen Vorrichtung, mit:
einem Leitungskörper,
einem leitenden Elektrodenschaft;
einem Spulenleiter, der mit dem leitenden Elektrodenschaft elektrisch gekoppelt ist; und
einer Spitzenelektrode (108), die mit dem leitenden Elektrodenschaft gekoppelt ist;
**gekennzeichnet durch**
einen Hochfrequenz-Umleitungshülsenkopf (HF-Hülsenkopf) (201), der mit dem leitenden Elektrodenschaft elektrisch gekoppelt ist; wobei der HF-Umleitungshülsenkopf enthält:
ein leitendes Element (202); und
eine biostabile dielektrische Beschichtung (204) für die Gewebekontaktierung, die über das leitende Element eingeführt wird.

2. Leitung einer medizinischen Vorrichtung nach Anspruch 1, wobei der Hülsenkopf dafür ausgelegt ist, Hochfrequenzsignale von der Spitzenelektrode und in den Hülsenkopf umzuleiten.

3. Leitung einer medizinischen Vorrichtung nach Anspruch 1, wobei ein Oberflächenbereich des leitenden Elements etwa zehnmal größer ist als ein Oberflächenbereich der Spitzenelektrode.

4. Leitung einer medizinischen Vorrichtung nach Anspruch 1, wobei das leitende Element am distalen Ende des leitenden Elements einen vergrößerten Durchmesser und am proximalen Ende des leitenden Elements einen verkleinerten Durchmesser aufweist.

5. Leitung einer medizinischen Vorrichtung nach Anspruch 3, die ferner eine leitende Abdichteinrichtung (212) enthält, die mit dem leitenden Element gekoppelt ist.

6. Leitung einer medizinischen Vorrichtung nach Anspruch 1, wobei die biostabile dielektrische Beschichtung Polyamid und/oder Parylen und/oder Metalloxide enthält.

7. Leitung einer medizinischen Vorrichtung nach Anspruch 1, wobei das leitende Element Titan und/oder eine Titanlegierung und/oder leitende Polymere enthält.

8. Leitung einer medizinischen Vorrichtung nach Anspruch 2, wobei wenigstens 80 % der HF-Leistung durch den HF-Hülsenkopf abgeleitet wird.

9. Leitung einer medizinischen Vorrichtung nach Anspruch 2, wobei wenigstens 70 % der HF-Leistung durch den HF-Hülsenkopf abgeleitet wird.

10. Leitung einer medizinischen Vorrichtung nach Anspruch 2, wobei wenigstens 60 % der HF-Leistung durch den HF-Hülsenkopf abgeleitet wird.

11. Leitung einer medizinischen Vorrichtung nach Anspruch 1, ferner mit einer leitenden Abdichteinrichtung (212), die den HF-Umleitungshülsenkopf mit dem leitenden Elektrodenschaft koppelt.

12. Leitung einer medizinischen Vorrichtung nach einem der Ansprüche 1 bis 11, ferner mit einem oder mehreren leitenden Ringen (1224), die den HF-Umleitungshülsenkopf mit dem leitenden Elektrodenschaft koppeln.

## Revendications

1. Fil de dispositif médical comportant :
un corps de fil,
un arbre d'électrode conductrice contenu dans le corps de fil ;
un conducteur de bobine électriquement couplé à l'arbre d'électrode conductrice ;
une électrode de pointe (108) couplée à l'arbre d'électrode conductrice ; et **caractérisé par**
une tête de manchon shuntée par radiofréquences (RF) (201) électriquement couplée à l'arbre d'électrode conductrice ;
et dans lequel la tête de manchon shuntée RF comprend :
un élément conducteur (202) ; et
un revêtement diélectrique à interfaçage de tissu biostable (204) introduit sur l'élément conducteur.

2. Fil de dispositif médical selon la revendication 1, dans lequel la tête de manchon est conçue pour shunter des signaux à haute fréquence à distance de l'électrode de pointe et dans la tête de manchon.

3. Fil de dispositif médical selon la revendication 1, dans lequel une zone de surface de l'élément conducteur est environ dix fois plus grande qu'une zone de surface de l'électrode de pointe.

4. Fil de dispositif médical selon la revendication 1, dans lequel l'élément conducteur comprend un diamètre augmenté au niveau de l'extrémité distale et un diamètre réduit au niveau de l'extrémité proximale de l'élément conducteur.

5. Fil de dispositif médical selon la revendication 3, comportant en outre un agent de scellement conducteur (212) couplé à l'élément conducteur.

6. Fil de dispositif médical selon la revendication 1, le revêtement diélectrique biostable étant au moins l'un parmi du polyamide, du parylène, et des oxydes métalliques.

7. Fil de dispositif médical selon la revendication 1, l'élément conducteur étant au moins l'un parmi du titane, un alliage de titane, et des polymères conducteurs.

8. Fil de dispositif médical selon la revendication 2, dans lequel au moins 80 % de la puissance RF est shuntée par la tête de manchon RF.

9. Fil de dispositif médical selon la revendication 2, dans lequel au moins 70 % de la puissance RF est shuntée par la tête de manchon RF.

10. Fil de dispositif médical selon la revendication 2, dans lequel au moins 60 % de la puissance RF est shuntée par la tête de manchon RF.

11. Fil de dispositif médical selon la revendication 1, comportant en outre un agent de scellement conducteur (212) qui couple la tête de manchon shuntée RF à l'arbre d'électrode conductrice.

12. Fil de dispositif médical selon l'une quelconque des revendications 1 à 11, comportant en outre un ou plusieurs anneaux conducteurs (1224) qui couplent la tête de manchon shuntée RF à l'arbre d'électrode conductrice.
